# EUROPÄISCHE PATENTSCHRIFT

(11) **EP 3 354 305 B1**
(45) Veröffentlichungstag und Bekanntmachung des Hinweises auf die Patenterteilung: **18.11.2020**
(21) Anmeldenummer: 18151041.3
(22) Anmeldetag: 10.01.2018
(51) Int. Cl.: A61M 5/162, A61M 5/14

(54) **ÜBERLEITUNGSSYSTEM FÜR EIN MEDIZINISCHES INFUSIONSSYSTEM**
TRANSFER SYSTEM FOR A MEDICAL INFUSION SYSTEM
SYSTÈME D'ÉCOULEMENT POUR UN SYSTÈME DE PERFUSION MÉDICALE

(30) Priorität: 20.01.2017 DE 102017200932
(43) Veröffentlichungstag der Anmeldung: 01.08.2018
(73) Patentinhaber: B. Braun Melsungen AG, 34212 Melsungen (DE)
(72) Erfinder: Bolz, Johannes, 34134 Kassel (DE); Schlitt, Christof, 34621 Obergrenzebach (DE); Vial, Siegbert, 35099 Burgwald (DE)
(74) Vertreter: Patentanwälte Ruff, Wilhelm, Beier, Dauster & Partner mbB

(56) Entgegenhaltungen:
- DD-A1- 269 558
- DE-U- 7 709 245
- DE-U1- 8 802 488
- DE-U1-202015 103 791
- FR-A1- 2 802 430
- GB-A- 809 427

## Beschreibung

Die Erfindung betrifft ein Überleitungssystem für ein medizinisches Infusionssystem mit mehreren Funktionsteilen, die ein Einstechteil mit einem Einstechdorn aufweisen, der einen Flüssigkeitskanal sowie einen Belüftungskanal aufweist, der in einen Belüftungsausgang zu einer Außenseite des Einstechteils hin mündet, der mit einem Luftfilterelement versehen ist.

Ein derartiges Überleitungssystem ist aus der EP 0 582 038 B1 bekannt. Das bekannte Überleitungssystem weist eine Tropfkammer auf, der in einem oberen Bereich ein Einstechteil mit einem Einstechdorn zugeordnet ist. In einem unteren Bereich ist die Tropfkammer mit einer Schlauchleitung verbunden, die in einen Katheter mündet, der mit einem Patienten verbindbar ist. Das Einstechteil ist mit einem Belüftungskanal versehen, der einen Einstechdorn durchdringt und zu einer Außenseite des Einstechteils in einen Belüftungsausgang mündet, dem ein Luftfilterelement zugeordnet ist. Dem Belüftungskanal ist zudem ein Rückschlagventil zugeordnet, das eine Belegung des Luftfilterelements mit Flüssigkeit verhindert, die ungewollt in den Belüftungskanal im Bereich des Einstechdorns eindringt. Weiterhin sind aus der DE 20 2015 103 791 U, der DE 77 09 245 U und der DD 269 558 A1 Überleitungssysteme gemäß dem Oberbegriff des Anspruchs 1 bekannt.

Aufgabe der Erfindung ist es, ein Überleitungssystem der eingangs genannten Art zu schaffen, das in kostengünstiger Weise sowohl eine belüftete als auch eine unbelüftete Infusionstherapie ermöglicht, einfach herzustellen und sicher in der Handhabung ist.

Diese Aufgabe wird dadurch gelöst, dass ein Verschlusselement für den Belüftungskanal vorgesehen ist, das als Abreißkörper an einem Funktionsteil angeformt ist. Das als Abreißkörper gestaltete Verschlusselement ermöglicht ein Absperren oder Freigeben des Belüftungskanals, so dass je nach Einsatz des Verschlusselements eine belüftete oder eine unbelüftete Infusionstherapie durchgeführt werden kann. Dadurch, dass das Verschlusselement als Abreißkörper an einem Funktionsteil des Überleitungssystems angeformt ist, ist das Verschlusselement gemeinsam mit dem Funktionsteil herstellbar, insbesondere in einem einstufigen Kunststoffspritzgussvorgang. Die Gestaltung als Abreißkörper ermöglicht ein Lösen des Verschlusselements von dem Funktionsteil. Geeignete Funktionsteile sind insbesondere ein Gehäuse des Einstechteils, ein Gehäuse eines Anschlussbereichs einer Tropfkammer an eine Schlauchleitung, eine Schutzkappe für den Einstechdorn oder Ähnliches. Vorzugsweise ist das Verschlusselement wie auch das zugehörige Funktionsteil aus einem thermoplastischen Kunststoff hergestellt.

Erfindungsgemäß ist als Funktionsteil eine Schutzkappe für den Einstechdorn vorgesehen, an der das Verschlusselement angeformt ist. Die Schutzkappe kann entweder axial auf den Einstechdorn aufsteckbar oder auf den Einstechdorn aufrastbar gestaltet sein.

In weiterer Ausgestaltung der Erfindung ist das Verschlusselement an der Schutzkappe derart positioniert, dass das Verschlusselement in einem auf dem Einstechdorn montierten Zustand der Schutzkappe über dem Belüftungsausgang positioniert ist. Dadurch kann das Verschlusselement in einfacher Weise auf den Belüftungsausgang gedrückt werden und gleichzeitig die Schutzkappe von dem Einstechdorn entfernt werden. Durch das Fixieren des Verschlusselements am Belüftungsausgang und das zeitgleiche Entfernen der Schutzkappe von dem Einstechdorn reißt das Verschlusselement zwangsläufig von der Schutzkappe ab.

In weiterer Ausgestaltung der Erfindung weist das Verschlusselement einen Verschlussstopfen auf, der bei auf dem Einstechdorn montiertem Zustand der Schutzkappe in fluchtender Ausrichtung zu dem Belüftungsausgang positioniert ist. Dadurch ist gewährleistet, dass der Verschlussstopfen durch eine einfache Axialbewegung, insbesondere durch eine manuelle Druckbelastung, in den Belüftungsausgang gedrückt werden kann, wodurch sich ein dichter Verschluss des Belüftungsausgangs ergibt. Der montierte Zustand der Schutzkappe kann durch Aufstecken, durch Aufrasten oder durch Ähnliches erzielt sein.

In weiterer Ausgestaltung der Erfindung sind das Verschlusselement und das Funktionsteil als gemeinsames Kunststoffbauteil in einem Spritzgussvorgang hergestellt. Dies ermöglicht eine einfache und kostengünstige Herstellung des Funktionsteils und des Verschlusselements in einem einzelnen Spritzgusswerkzeug.

In weiterer Ausgestaltung der Erfindung ist das Verschlusselement an einem Umfangsrand der Schutzkappe angeformt. Vorzugsweise ragt das Verschlusselement vom Umfangsrand der Schutzkappe aus zumindest weitgehend radial nach außen ab - auf eine Mittellängsachse der Schutzkappe bezogen. Das Verschlusselement ist vorzugsweise mittels eines Abrissstegs an der Schutzkappe angeformt, der radial nach außen von dem Umfangsrand der Schutzkappe abragt.

In weiterer Ausgestaltung der Erfindung weist der Abrisssteg eine Solltrennstelle auf, die ein Lösen des Verschlusselements von dem Umfangsrand der Verschlusskappe bei einer relativen Drehbewegung in Umfangsrichtung zwischen Schutzkappe und Verschlusselement bewirkt. Sobald daher das Verschlusselement manuell relativ zu dem Einstechteil fixiert und gleichzeitig die Schutzkappe von dem Einstechdorn gelöst wird, insbesondere durch eine Abziehbewegung, reißt das Verschlusselement von der Schutzkappe ab. Die Solltrennstelle ist derart gestaltet, dass sie eine Kerbfunktion aufweist, die durch die relative Zugbelastung zwischen Schutzkappe und Verschlusselement aktiviert wird.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch eine Ausführungsform eines erfindungsgemäßen Überleitungssystems als Teil eines medizinischen Infusionssystems,
- Fig. 2a: schematisch das Überleitungssystem nach Fig. 1 mit einer Schutzkappe auf einem Einstechdorn,
- Fig. 2b: einen Teilbereich des Überleitungssystems nach Fig. 2a in schematischer Längssch nittdarstellung,
- Fig. 3a: das Überleitungssystem nach Fig. 2a mit entfernter Schutzkappe,
- Fig. 3b: schematisch in vergrößerter Längsschnittdarstellung analog Fig. 2b das Überleitungssystem nach Fig. 3a,
- Fig. 4: in schematischer Schnittdarstellung eine weitere Ausführungsform eines erfindungsgemäßen Überleitungssystems im Bereich eines Einstechteils mit aufgesteckter Schutzkappe und
- Fig. 5: die Schutzkappe gemäß Fig. 4 mit angeformtem Verschlusselement für einen Belüftungsausgang des Einstechteils nach Fig. 4.

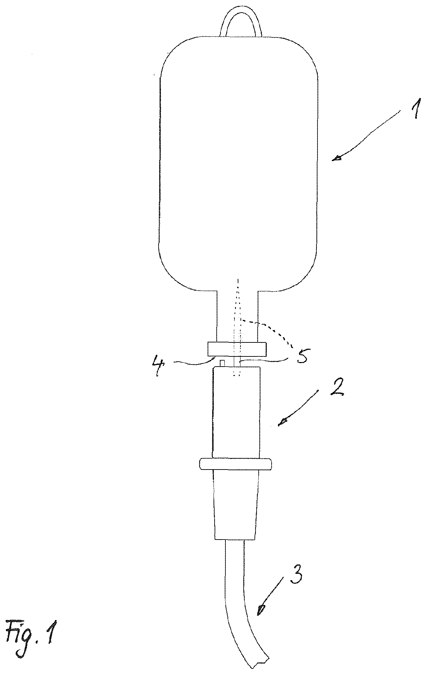

Ein medizinisches Infusionssystem gemäß Fig. 1 weist ein Infusionsbehältnis 1 auf, das als Speicher für eine Infusionsflüssigkeit dient, die für eine entsprechende medizinische Infusionstherapie verwendet wird. Das Infusionsbehältnis 1 ist in betriebsfertigem Funktionszustand an einem nicht dargestellten Haken eines Ständers oder eines Trägers aufgehängt. Im Bereich einer Unterseite ist das Infusionsbehältnis mit einem Anschlussbereich 4 versehen, der einen nicht näher dargestellten, elastisch nachgiebigen Verschlussstopfen aufweist. Mit dem Anschlussbereich 4 verbunden ist ein Überleitungssystem 2, das als Tropfkammer ausgeführt ist. Die Tropfkammer ist im Bereich ihrer Oberseite mit einem Einstechteil versehen, das einen Einstechdorn 5 umfasst. Im Bereich einer Unterseite ist ein Tropfkammerboden der Tropfkammer mit einer Schlauchleitung 3 verbunden, die ebenfalls Teil des Infusionssystems ist.

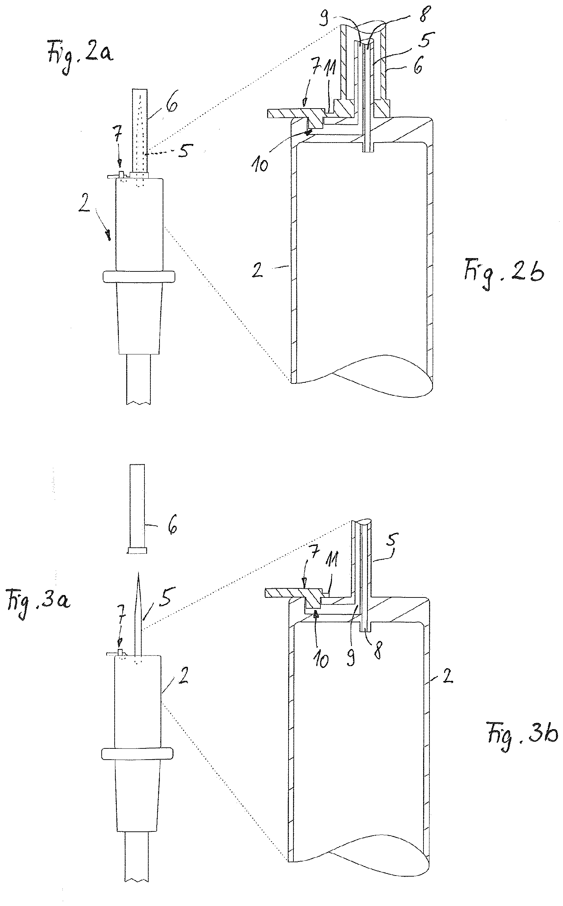

Das Einstechteil bildet ein Gehäuse im Bereich einer Oberseite der Tropfkammer. Das Gehäuse setzt sich in den nach oben abragenden Einstechdorn 5 fort, der mit seiner Spitze in betriebsfertig montiertem Zustand durch den elastisch nachgiebigen Verschlussstopfen des Anschlussbereichs 4 des Infusionsbehältnisses 1 hindurchgestochen ist. Die Länge des Einstechdorns 5 ist größer als eine Dicke des Verschlussstopfens, so dass eine Spitze des Einstechdorns 5 axial über den Verschlussstopfen hinaus ins Innere des Infusionsbehältnisses 1 hineinragt. In dem Einstechdorn 5 erstreckt sich zum einen ein Flüssigkeitskanal 8 und zum anderen ein parallel neben diesem verlaufender Belüftungskanal 9. Der Belüftungskanal 9 setzt sich in dem Gehäuse des Einstechteils in einen Belüftungsausgang 10 zu einer Außenseite des Gehäuses des Einstechteils fort, wodurch der Belüftungskanal 9 über den Belüftungsausgang 10 zu einer Umgebung hin offen ist (siehe Fig. 2a bis 3b). Dem Belüftungsausgang 10 ist in nicht näher dargestellter Weise ein Luftfilterelement zugeordnet, um zu gewährleisten, dass ausschließlich gefilterte Luft über den Belüftungsausgang 10 durch den Belüftungskanal 9 hindurch in das Infusionsbehältnis 1 eindringen kann. Mittels des Belüftungskanals 9 ist eine belüftete Infusionstherapie bei dem Infusionssystem gemäß Fig. 1 möglich.

Alternativ ist es möglich, den Belüftungsausgang 10 zu verschließen mittels eines Verschlusselements 7. Das Verschlusselement 7 ist derart auf den Belüftungsausgang 10 abgestimmt, dass das Verschlusselement 7 den Belüftungsausgang 10 dicht verschließen kann, wenn das Verschlusselement 7 in den Belüftungsausgang 10 eingesetzt ist.

Das Verschlusselement 7 wird gemeinsam mit einer Schutzkappe 6 für den Einstechdorn 5 hergestellt. Sowohl die Schutzkappe 6 als auch das Verschlusselement 7 sind aus demselben Kunststoffmaterial in einem gemeinsamen Spritzgussverfahren als einteiliges Bauteil hergestellt. Wie anhand der Fig. 2b erkennbar ist, ist das Verschlusselement 7 im Bereich eines unteren Umfangsrands der Schutzkappe 6 über einen Abrisssteg 11 mit der Schutzkappe 6 verbunden. Der Abrisssteg 11 definiert eine Solltrennlinie zwischen dem Verschlusselement 7 und dem Umfangsrand der Schutzkappe 6, so dass bei einer kräftigen Relativbewegung zwischen der Schutzkappe 6 und dem Verschlusselement 7 das Verschlusselement 7 im Bereich des Abrissstegs 11 von der Schutzkappe 6 abreißt. Der Abrisssteg 11 ist dünnwandig und schmal ausgeführt. Der Abrisssteg 11 ragt relativ zu einer Mittellängsachse der Schutzkappe 6 im Bereich des Umfangsrands radial nach außen ab. Auch das an dem Abrisssteg 11 angeformte Verschlusselement 7 ragt seitlich radial nach außen ab in Verlängerung des Abrissstegs 11.

Anhand der Fig. 2a und 2b ist erkennbar, dass das Verschlusselement 7 bei auf dem Einstechdorn 5 montierter Schutzkappe 6 zwangsläufig so ausgerichtet ist, dass ein Verschlussstopfen des Verschlusselements 7 in den Belüftungsausgang 10 hineinragt. Wenn nun das Verschlusselement 7 manuell nach unten gegen den Belüftungsausgang 10 gedrückt wird, wodurch der Verschlussstopfen dicht in den Belüftungsausgang 10 gepresst wird, und gleichzeitig die Schutzkappe 6 auf dem Einstechdorn 5 nach oben abgezogen wird, dann reißt zwangsläufig der Abrisssteg 11 ab, während das Verschlusselement 7 in seiner Verschlussposition auf dem Belüftungsausgang 10 des Belüftungskanals 9 verbleibt.

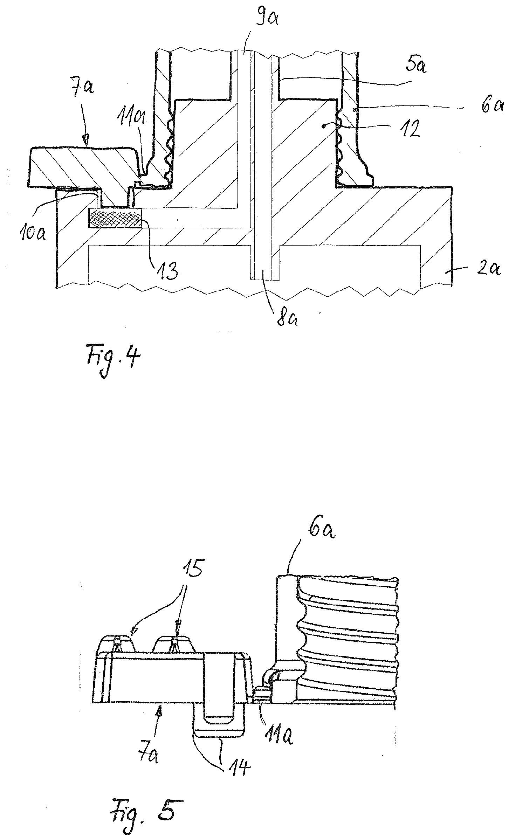

Die Ausführungsform gemäß den Fig. 4 und 5 entspricht im Wesentlichen der zuvor anhand der Fig. 1 bis 3b beschriebenen Ausführungsform. Funktionsgleiche Bauteile oder Abschnitte sind demzufolge mit gleichen Bezugszeichen unter Hinzufügung des Buchstabens a versehen. Zur Vermeidung von Wiederholungen wird nachfolgend lediglich auf die Unterschiede der Ausführungsform nach den Fig. 4 und 5 relativ zur Ausführungsform nach den Fig. 1 bis 3b eingegangen.

Die Schutzkappe 6a ist auf einem Haltesockel 12 des Einstechteils der Tropfkammer 2a durch Aufstecken als Presssitz montiert. Die Schutzkappe 6a weist im Bereich ihres unteren Umfangsrands innenseitig eine Umfangsprofilierung in Form eines Innengewinde auf, das Belüftungskanäle relativ zu einem Außenmantel des Haltesockels 12 bildet, um eine sichere Sterilisation des Infusionssystems bei aufgesteckter Schutzkappe 6a zu ermöglichen. Die Darstellung anhand der Fig. 4 ist lediglich schematisch, die Darstellung gemäß Fig. 5 stellt eine detaillierte Skizzierung der Ausführungsform nach Fig. 4 dar.

Der Verschlussstopfen 7a ist außenseitig im Bereich des Umfangsrands der Schutzkappe 6a mittels eines Abrissstegs 11a angeformt. Dem Belüftungsausgang 10a des Belüftungskanals 9a ist ein Luftfilterelement 13 zugeordnet, das anhand der Fig. 4 schematisch dargestellt ist. Wie Fig. 4 und insbesondere Fig. 5 zu entnehmen ist, weist das einstückig an der Schutzkappe 6a angeformte Verschlusselement 7a einen Verschlussstopfen 14 auf, der dicht in den Belüftungsausgang 10a einpassbar ist. Das Verschlusselement 7a weist zudem im Bereich seiner Oberseite Griffprofilierungen 15 auf, die eine Handhabung des Verschlusselements 7a erleichtern. Das Verschlusselement 7a ist - relativ zu einer Mittellängsachse der Schutzkappe 6a - radial mit einem Abrisssteg 11a verbunden, der von einer Außenseite des Umfangsrands der Schutzkappe 6a radial nach außen abragt. Der Abrisssteg 11a bildet eine Solltrennlinie und weist eine entsprechende Kerbfunktion auf, um bei einer Relativbewegung zwischen dem Verschlusselement 7a und der Schutzkappe 6a ein Abreißen des Verschlusselements 7a zu ermöglichen. Eine Trennung zwischen dem Verschlusselement 7a und der Schutzkappe 6a erfolgt in diesem Fall im Bereich des Abrissstegs 11a. Das Verschlusselement 7a bildet - wie auch das Verschlusselement 7 bei der Ausführungsform gemäß den Fig. 1 bis 3b - demzufolge einen Abrisskörper im Sinne der Erfindung.

Die Verschlusskappe 6a wird zur Montage auf dem Einstechdorn 5a axial von oben her auf den Einstechdorn 5a kraftschlüssig aufgesteckt. Die Ausrichtung bei einem axialen Aufsteckvorgang erfolgt so, dass das Verschlusselement 7a mit seinem Verschlussstopfen 14 axial über dem Belüftungsausgang 10a positioniert ist und in den Belüftungsausgang 10a hineingedrückt werden kann. Sobald nun das Verschlusselement 7a in seiner den Belüftungsausgang 10a verschließenden Verschlussstellung durch manuellen Druck von oben her gehalten wird und gleichzeitig die Schutzkappe 6a nach oben abgezogen, um diese von dem Haltesockel 12 des Einstechteils axial zu entfernen, reißt zwangsläufig der Abrisssteg 11a ab und trennt die Verbindung zwischen dem Verschlusselement 7a und der Schutzkappe 6a. Ein manueller Kraftaufwand durch Ziehen der Schutzkappe 6a reicht aus, um die Trennung im Bereich des Abrissstegs 11 zu erreichen. Damit verbleibt das Verschlusselement 7a in seiner den Belüftungskanal 9a verschließenden Verschlussstellung, wodurch eine unbelüftete Infusionstherapie durchgeführt werden kann, sobald der Einstechdorn 5a gemäß Fig. 1 in den Anschlussbereich 4 eingestochen ist. Soll eine belüftete Therapie erfolgen, kann das Verschlusselement 7a entweder nachträglich von dem Belüftungsausgang 10a entfernt werden, wodurch der Belüftungsausgang 10a und demzufolge der Belüftungskanal 9a frei sind. Alternativ kann das Verschlusselement 7a über den Abrisssteg 11a mit der Schutzkappe 6a verbunden bleiben und erst nach dem Lösen der Schutzkappe 6a von der Schutzkappe 6a getrennt werden.

## Patentansprüche

1. Überleitungssystem für ein medizinisches Infusionssystem mit mehreren Funktionsteilen, die ein Einstechteil mit einem Einstechdorn (5, 5a) aufweisen, der einen Flüssigkeitskanal (8, 8a) sowie einen Belüftungskanal (9, 9a) aufweist, der in einen Belüftungsausgang (10, 10a) zu einer Außenseite des Einstechteils hin mündet, der mit einem Luftfilterelement (13) versehen ist, wobei ein Verschlusselement (7, 7a) für den Belüftungskanal (9, 9a) vorgesehen ist, das als Abreißkörper an einem Funktionsteil angeformt ist, **dadurch gekennzeichnet, dass** dieses Funktionsteil als eine Schutzkappe (6, 6a) für den Einstechdorn (5, 5a) vorgesehen ist, an der das Verschlusselement (7, 7a) angeformt ist.

2. Überleitungssystem nach Anspruch 1, **dadurch gekennzeichnet, dass** das Verschlusselement (7, 7a) an der Schutzkappe (6, 6a) derart positioniert ist, dass das Verschlusselement (7, 7a) in einem auf dem Einstechdorn (5, 5a) montierten Zustand der Schutzkappe (6, 6a) über dem Belüftungsausgang (10, 10a) positioniert ist.

3. Überleitungssystem nach einem der vorhergehenden Ansprüche, **dadurch gekennzeichnet, dass** das Verschlusselement (7a) einen Verschlussstopfen (14) aufweist, der bei auf dem Einstechdorn (5a) montiertem Zustand der Schutzkappe (6a) in fluchtender Ausrichtung zu dem Belüftungsausgang (10a) positioniert ist.

4. Überleitungssystem nach einem der vorhergehenden Ansprüche, **dadurch gekennzeichnet, dass** das Verschlusselement (7, 7a) und das Funktionsteil als gemeinsames Kunststoffbauteil in einem Spritzgussvorgang hergestellt sind.

5. Überleitungssystem nach einem der vorhergehenden Ansprüche, **dadurch gekennzeichnet, dass** das Verschlusselement (7, 7a) an einem Umfangsrand der Schutzkappe (6, 6a) angeformt ist.

6. Überleitungssystem nach Anspruch 5, **dadurch gekennzeichnet, dass** das Verschlusselement (7, 7a) mittels eines Abrissstegs (11, 11a) radial zu einer Längsachse der Schutzkappe (6, 6a) seitlich nach außen abragt.

7. Überleitungssystem nach Anspruch 6, **dadurch gekennzeichnet, dass** der Abrisssteg (11, 11a) eine Solltrennstelle aufweist, die ein Lösen des Verschlusselements (7, 7a) von dem Umfangsrand der Schutzkappe (6, 6a) bei einer relativen Drehbewegung in Umfangsrichtung zwischen Schutzkappe (6a) und Verschlusselement (7a) bewirkt.

## Claims

1. Transfer system for a medical infusion system having multiple functional parts which include a piercing part with a piercing mandrel (5, 5a), which includes a fluid channel (8, 8a) and a ventilation channel (9, 9a) ending towards a ventilation outlet (10, 10a) to an outside of the piercing part, which is provided with an air filter element (13), wherein a closure element (7, 7a) is provided for the ventilation channel (9, 9a) and moulded on a functional part in the form of a tear-off body,
**characterized in that**
this functional part is provided in the form of a protection cap (6, 6a) for the piercing mandrel (5, 5a), to which cap the closure element (7, 7a) is moulded on.

2. Transfer system according to claim 1, **characterized in that** the closure element (7, 7a) is positioned on the protection cap (6, 6a) in such a manner that the closure element (7, 7a) is positioned above the ventilation outlet (10, 10a) in an assembled state of the protection cap (6, 6a) on the piercing mandrel (5, 5a).

3. Transfer system according to any of the preceding claims, **characterized in that** the closure element (7a) has a closure plug (14) which is positioned in aligned orientation relative to the ventilation outlet (10a) in the assembled state of the protection cap (6a) on the piercing mandrel (5a).

4. Transfer system according to any of the preceding claims, **characterized in that** the closure element (7, 7a) and the functional part are produced in an injection moulding procedure as a common plastic component.

5. Transfer system according to any of the preceding claims, **characterized in that** the closure element (7, 7a) is moulded on a peripheral edge of the protection cap (6, 6a).

6. Transfer system according to claim 5, **characterized in that** the closure element (7, 7a) projects laterally outwards radial to a longitudinal axis of the protection cap (6, 6a) by means of a tear-off web (11, 11a).

7. Transfer system according to claim 6, **characterized in that** the tear-off web (11, 11a) has a predetermined separation point which causes disengagement of the closure element (7, 7a) from the peripheral edge of the protection cap (6, 6a) upon a relative turning movement in the circumferential direction between protection cap (6a) and closure element (7a).

## Revendications

1. Système de transfert pour un système de perfusion médical comprenant plusieurs pièces fonctionnelles qui présentent une pièce de perforation avec un poinçon de perforation (5, 5a) qui présente un canal de liquide (8, 8a) ainsi qu'un canal de ventilation (9, 9a) qui débouche dans une sortie de ventilation (10, 10a) vers un côté extérieur de la pièce de perforation qui est pourvue d'un élément de filtre à air (13), un élément de fermeture (7, 7a) pour le canal de ventilation (9, 9a) étant prévu, lequel est formé en tant que corps déchirable au niveau d'une pièce fonctionnelle, **caractérisé en ce que** cette pièce fonctionnelle est prévue sous forme de capuchon de protection (6, 6a) pour le poinçon de perforation (5, 5a), sur lequel est façonné l'élément de fermeture (7, 7a).

2. Système de transfert selon la revendication 1, **caractérisé en ce que** l'élément de fermeture (7, 7a) est positionné sur le capuchon de protection (6, 6a) de telle sorte que l'élément de fermeture (7, 7a) soit positionné par-dessus la sortie de ventilation (10, 10a) dans un état du capuchon de protection (6, 6a) monté sur le poinçon de perforation (5, 5a).

3. Système de transfert selon l'une quelconque des revendications précédentes, **caractérisé en ce que** l'élément de fermeture (7a) présente un bouchon de fermeture (14), qui, dans l'état du capuchon de protection (6a) monté sur le poinçon de perforation (5a), est positionné en alignement avec la sortie de ventilation (10a).

4. Système de transfert selon l'une quelconque des revendications précédentes, **caractérisé en ce que** l'élément de fermeture (7, 7a) et la pièce fonctionnelle sont fabriqués sous forme de composant en plastique commun dans une opération de moulage par injection.

5. Système de transfert selon l'une quelconque des revendications précédentes, **caractérisé en ce que** l'élément de fermeture (7, 7a) est façonné au niveau d'un bord périphérique du capuchon de protection (6, 6a).

6. Système de transfert selon la revendication 5, **caractérisé en ce que** l'élément de fermeture (7, 7a) fait saillie latéralement vers l'extérieur au moyen d'une nervure d'arrachage (11, 11a) radialement par rapport à un axe longitudinal du capuchon de protection (6, 6a).

7. Système de transfert selon la revendication 6, **caractérisé en ce que** la nervure d'arrachage (11, 11a) présente une zone destinée à la séparation qui provoque un détachement de l'élément de fermeture (7, 7a) du bord périphérique du capuchon de protection (6, 6a) lors d'un mouvement de rotation relatif dans la direction périphérique entre le capuchon de protection (6a) et l'élément de fermeture (7a).
